# EUROPEAN PATENT APPLICATION

(11) **EP 2 497 423 A1**
(43) Date of publication of application: **12.09.2012**
(21) Application number: 12158000.5
(22) Date of filing: 05.03.2012
(51) Int. Cl.: A61B 5/22

(54) **Biometric apparatus**

(30) Priority: 07.03.2011 JP 2011049142
(71) Applicant: Tanita Corporation, Tokyo (JP)
(72) Inventor: Shiota, Takayuki, Itabashi-ku, Tokyo (JP); Sato, Tomio, Itabashi-ku, Tokyo (JP)
(74) Representative: Grünecker, Kinkeldey, Stockmair & Schwanhäusser

(57) **Abstract**

A biometric apparatus includes an instruction issuing unit configured to issue an instruction to an object person to be measured to execute a predetermined state; a pulse sensor configured to measure a pulse rate in the predetermined state; a consumed energy measuring unit configured to measure a consumed energy in the predetermined state; and a biological data calculating unit configured to define a relational expression of the consumed energy with respect to a predetermined pulse rate of the object person to be measured on the basis of the pulse rate measured by the pulse sensor in the predetermined state and the consumed energy measured by the consumed energy measuring unit at the measured pulse rate, and calculate biological data of the object person to be measured on the basis of the defined relational expression.

## Description

### BACKGROUND OF THE INVENTION

### 1. Field of the Invention

The present invention relates to a biometric apparatus.

### 2. Description of the Related art

In the related art, when determining physical strength of an object person to be measured, the physical strength is generally determined on the basis of how much endurance the object person to be measured has. Generally, the larger a value VO₂max (the maximum oxygen uptake per unit time), the better the endurance is evaluated to be. Therefore, accurate measurement of the value VO₂max is important when determining the physical strength of the object person to be measured.

JP-A-2006-238970 is an example of the related art.

However, the accurate measurement of the value VO₂max puts an enormous load on the object person to be measured. In other words, since the value VO₂max is needed to be obtained by applying an exercise stress, which is extremely close to the limit for the object person to be measured, that is, which causes a heart rate to be a value near the maximum heart rate, to the object person to be measured, measuring a value VO₂ (oxygen uptake) during the exercise, and considering the obtained value as the value VO₂max, the load applied to the object person to be measured is significantly large. Also, since such a measurement requires a large scale of apparatus or equipment, the object person to be measured is obliged to go to a specific site where the apparatus or equipment are provided for the measurement, which is inconvenient for the object person to be measured.

In contrast, the value VO₂ at the maximum heart rate (that is, VO₂(HRmax)) may be considered to be close to the value VO₂max. The value VO₂ has a high correlation with a consumed energy and, simultaneously, the value VO₂(HRmax has a high correlation with a consumed energy (EE(75%HRmax)) at a heart rate which is 75% of the maximum heart rate. In view of this point, the inventors have developed a biometric apparatus which is capable of determining a relational expression of the consumed energy with respect to a predetermined pulse rate of the object person to be measured with a simple measurement. Although the heart rate and a pulse rate are basically synonymous terms, in the following description, the term "heart rate" is used to mean the number of pulses of heart, and the term "pulse rate" is used to mean the number of pulses of peripheral organ, respectively.

### SUMMARY OF THE INVENTION

It is an object of the invention to provide a biometric apparatus which is capable of obtaining a relational expression of a consumed energy with respect to a predetermined pulse rate of an object person to be measured without applying an excessive load to the object person to be measured with simple measurement.

In order to solve the above-described problem, there is provided a biometric apparatus including: an instruction issuing unit configured to issue an instruction to an object person to be measured to execute a predetermined state; a pulse sensor configured to measure a pulse rate in the predetermined state; a consumed energy measuring unit configured to measure a consumed energy in the predetermined state; and a biological data calculating unit configured to define a relational expression of the consumed energy with respect to a predetermined pulse rate of the object person to be measured on the basis of the pulse rate measured by the pulse sensor in the predetermined state and the consumed energy measured by the consumed energy measuring unit at the pulse rate, and calculate biological data of the object person to be measured on the basis of the relational expression.

Preferably, the instruction issuing unit issues an instruction to execute at least a first state, a second state which is a state of action applying a load larger than that of the first state, and a third state which is a state of action applying a load larger than that of the second state in sequence as the predetermined state, and the biological data calculating unit defines the relational expression by acquiring an approximated straight line on the basis of three points, namely, a pulse rate and a consumed energy of the object person to be measured in the first state, a pulse rate and a consumed energy of the object person to be measured in the second state, and a pulse rate and a consumed energy of the object person to be measured in the third state.

Preferably, if the difference between the pulse rate of the object person to be measured in the first state and the pulse rate of the object person to be measured in the second state is a predetermined threshold value or smaller, the instruction issuing unit issues an instruction to execute a state of action applying a load larger than that applied in the state of action to be instructed if the difference is larger than the predetermined threshold value as the third state.

Preferably, the biometric apparatus further includes a data input unit configured to be capable of entering data such as age, sex, body weight, and fat free mass of the object person to be measured, and the consumed energy measuring unit includes an acceleration sensor configured to be capable of detecting an acceleration value of a body movement of the object person to be measured, and calculates the consumed energy on the basis of the acceleration value of the body movement detected by the acceleration sensor and the data such as age, sex, body weight, and fat free mass entered by the data input unit.

Preferably, if the fat free mass entered by the data input unit is larger than a predetermined reference value, the instruction issuing unit issues an instruction to execute a state of action applying a load larger than that of the state of action to be instructed if the fat free mass is a predetermined reference value or smaller as the second state and/or the third state.

Preferably, if the fact that the change of the pulse rate of the object person to be measured in the predetermined state falls within a predetermined range is detected during the period of execution in which the predetermined state is continuously executed, the instruction issuing unit issues an instruction to terminate the execution of the predetermined state before the elapse of the period of execution, and if the fact is not detected, the instruction issuing unit issues an instruction to terminate the execution of the predetermined state after the period of execution has elapsed.

Preferably, the instruction issuing unit issues an instruction to execute a state of action executed continuously for a predetermined period in the same content as the predetermined state, and the biological data calculating unit defines the relational expression by acquiring the approximated straight line on the basis of the pulse rate and the consumed energy of the object person to be measured in the predetermined state measured by a plurality of times during the predetermined period.

Preferably, the biological data calculating unit calculates a value EE(75WHRmax) of the object person to be measured on the basis of the relational expression as the biological data, and calculates a value VO₂(HRmax) of the object person to be measured by applying the value EE(75%HRmax) to an expression expressing a correlation between the value EE(75%HRmax) and the value VO₂(HRmax). For reference sake, the value EE(75%HRmax) means a consumed energy at a heart rate (pulse rate) which is 75% of the maximum heart rate (pulse rate), and the value VO₂(HRmax) means a value VO₂ at the maximum heart rate (pulse rate) . The value VO₂ means an oxygen uptake.

Preferably, the biological data calculating unit determines the physical strength of the object person to be measured from the value of VO₂(HRmax of the object person to be measured, the sex and the age of the object person to be measured, and displays the result of determination on a display unit.

Preferably, the biological data calculating unit calculates the consumed energy according to the pulse rate by applying the pulse rate of the object person to be measured acquired by the pulse sensor to the relational expression as the biological data.

### Advantages of the Invention

According to the invention, the relational expression of the consumed energy with respect to the predetermined pulse rate of an object person to be measured may be obtained without applying an excessive load to the object person to be measured with simple measurement. By obtaining the consumed energy (EE(75%HRmax)) at a heart rate (pulse rate) which is 75% of the maximum heart rate (pulse rate) on the basis of the relational expression, VO₂(HRmax) which may be an index of the determination of the physical strength can be calculated, and hence the measurement of the physical strength without applying a large load to the object person to be measured is enabled.

Since the reduction in the size of the apparatus is achieved in this configuration, a large scale of apparatus or equipment is not necessary, and the simple measurement may be performed. In addition, since the relational expression of the consumed energy with respect to the predetermined pulse rate determined by the invention is defined on the basis of data specifically on the corresponding object person to be measured, acquisition of a relational expression with high degree of accuracy is enabled.

In addition, on the basis of the relational expression, only by measuring the pulse rate of the object person to be measured, the consumed energy with the measured pulse rate may be calculated. Therefore, in the state of action which may not be calculated accurately by a method of obtaining the magnitude of the acceleration value of the body movement by the acceleration sensor and calculating the consumed energy as in the related art (for example, in the state of operating a bicycle or climbing a mountain), the consumed energy can be calculated accurately by measuring the pulse rate at the corresponding period. Since both of the pulse sensor and the consumed energy calculating unit (the acceleration sensor) are provided, if the measurement of the pulse by the pulse sensor is performed, the consumed energy may be obtained as described above on the basis of the pulse rate, and if the measurement of the pulse by the pulse sensor is not performed, the consumed energy may be obtained from the acceleration value of the body movement as in the related art.

### BRIEF DESCRIPTION OF THE DRAWINGS

Fig. 1 is a block diagram showing a configuration of a biometric apparatus according to the invention;
Fig. 2 is a flowchart showing a flow of measurement of physical strength according to a first embodiment of the invention;
Fig. 3 is a graph showing a relationship between a pulse rate and a consumed energy;
Fig. 4 is a graph showing the relationship between the pulse rate and the consumed energy;
Fig. 5 is a graph showing the relationship between the pulse rate and the consumed energy;
Fig. 6 is a flowchart showing a flow of measurement of physical strength according to a second embodiment of the invention;
Fig. 7 is a flowchart showing a flow of measurement of physical strength according to a third embodiment of the invention; and
Fig. 8 is a graph showing the relationship between the pulse rate and the consumed energy;

### MODES FOR CARRYING OUT THE INVENTION

Referring now to the drawings, biometric measurement according to embodiments of the invention will be described in detail.

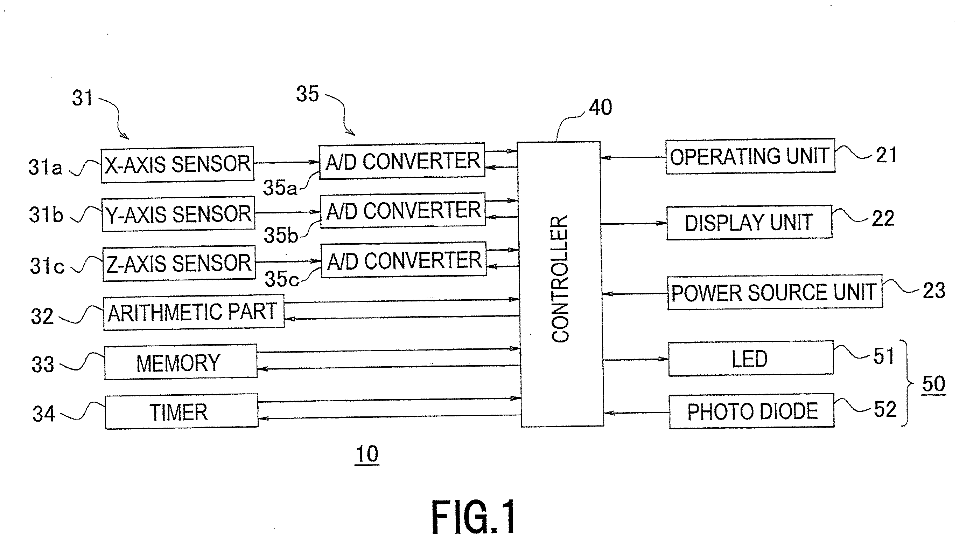

Fig. 1 is a block diagram showing a configuration of a biometric apparatus 10. As shown in Fig. 1, the biometric apparatus 10 includes an operating unit 21, a display unit 22, a power source unit 23, an acceleration sensor 31, an arithmetic part 32, a memory 33, a timer 34, an A/D converter 35, a controller 40, and a pulse sensor 50. Detailed configurations of the respective members will be described below.

The operating unit 21 functions mainly as a data input unit configured to allow a user to enter biological data of an object person to be measured or to enter set items of the biometric apparatus 10. The number, the shape, and an operating method of the operating unit 21 are not specifically limited, and may be selected as needed from, for example, those of a push-button type, a touch sensor type, and a dial type. Examples of the biological data to be entered by the operating unit 21 include age, sex, body weight, height, and fat free mass. However, the biometrical data are not specifically limited as long as they are needed for calculation of energy consumed by an action of the object person to be measured or measurement of physical strength as described later. Examples of the set items include set items required when the object person to be measured uses the biometric apparatus 10 and, for example, initial settings of the biometric apparatus 10, current time of the day and the day of week, and changeover of contents to be displayed on the display unit 22. The biological data and the set items entered in this manner are stored in the memory 33 under control of the controller 40, and are displayed on the display unit 22.

The display unit 22 is a display unit configured to display data sent from the controller 40, and mainly displays the biological data of the object person to be measured, the set items, an operation guide, an instruction to execute the predetermined state, current time of the day, date, and day of the week, measured pulse rate, calculated consumed energy, and determined result of the physical strength. The contents to be displayed are stored in the memory 33, and the controller 40 is configured to read out data from the memory 33 corresponding to the state of usage of the biometric apparatus 10 in accordance with a program stored in the memory 33 in advance, and display the same on the display unit 22. Examples of the display unit 22 include a display device using liquid crystal or an organic EL element may be employed, and the display unit 22 and the operating unit 21 may be formed integrally as a liquid crystal panel having, for example, a touch panel function.

The power source unit 23 is a power supply unit including a power supply source such as a battery, so that power is supplied to the respective components of the biometric apparatus 10 via the controller 40.

The memory 33 is a storage unit made up of a non-volatile memory using, for example, a semiconductor element. A combination of a volatile memory and the non-volatile memory may also be applicable. The volatile memory is configured to be able to store a variety of data for the processes by the controller 40 temporarily and, for example, functions as a temporary memory area used at the time of computing process of the arithmetic part 32. The non-volatile memory is used for storing data which is to be stored for a long time and, for example, is used for storing the biological data (age, sex, height, and so on) entered by the object person to be measured, a consumed energy calculating expression, a relational expression of the consumed energy with respect to a predetermined pulse rate (described later), an expression indicating a correlation between a value EE(75%HRmax) and a value VO₂(HRmax (described later), a determination table (described later), a variety of programs, and the like.

The timer 34 is a unit configured to count elapse of time (for example, a period of execution of a predetermined state and a predetermined period described later). In this embodiment, the timer 34 is an independent component. However, it may be integrated into the controller 40 as a timer circuit to determine whether or not the predetermined time has elapsed by the controller 40 by itself.

The acceleration sensor 31 is a sensor whose output value varies with an acceleration value generated by a body movement of the object person to be measured wearing the biometric apparatus 10, and is one of components of a consumed energy measuring unit. More specifically, the acceleration sensor 31 includes an X-axis sensor 31a, a Y-axis sensor 31b, and a Z-axis sensor 31c (see Fig. 1) so as to be capable of detecting the body movement in the 3-axes (X-axis, Y-axis, and Z-axis) directions orthogonal to each other, and is configured to be able to acquire an acceleration value, which is a value obtained by combining respective output values from the X-axis sensor 31a, the Y-axis sensor 31b, and the Z-axis sensor 31c.

The output acquired by the acceleration sensor 31 is converted from analogue to digital by the A/D converter 35 for the processes performed by the controller 40 or the arithmetic part 32. More specifically, the respective output values as analogue data acquired by the X-axis sensor 31a, the Y-axis sensor 31b, and the Z-axis sensor 31c are converted into digital data respectively by an A/D converter 35a, an A/D converter 35b, and an A/D converter 35c. The respective converted data may be stored in the memory 33 as body movement data in correspondence with an elapsed time from the start of acquisition in conjunction with the timer 34.

A/D conversion values of the respective output values from the X-axis sensor 31a, the Y-axis sensor 31b, and the Z-axis sensor 31c are combined by the arithmetic part 32. Accordingly, an acceleration value as digital data (A/D conversion value of the acceleration value) is calculated and is stored in the memory 33 as the body movement data in correspondence with the elapsed time from the start of acquisition in conjunction with the timer 34. The acceleration value may be stored as an integrated value of the acceleration value per predetermined period (the magnitude of the acceleration value). In this manner, by acquiring the acceleration value in correspondence with the elapsed time, not only the body movement strength, but also the presence or absence of the repetitiveness or continuity of the body movement, the pitch or the number of times (for example, the number of steps) of the body movement when the same body movement is repeated may be acquired simultaneously as the body movement data by chronologically observing the acceleration values according to the order of acquisition.

For reference sake, in order to acquire the acceleration value of any body movement of the object person to be measured (body movement data) by the acceleration sensor 31 further accurately, the biometric apparatus 10 is preferably worn by the object person to be measured in tight contact with the body of the object person to be measured. The acceleration value acquired in this manner is stored in the memory 33 and is partly (for example, the number of steps) displayed on the display unit 22 by the control of the controller 40.

The pulse sensor 50 includes an LED (light-emitting diode) 51 and a photo diode 52. The LED 51 irradiates a predetermined portion of the object person to be measured with a light beam having a wavelength suitable for measurement of the pulse, for example, a blue light beam. The light beam emitted from the LED 51 is absorbed by hemoglobin in a blood vessel of the object person to be measured and, simultaneously, is reflected by a subcutaneous tissue or the like, and is received by the photo diode 52. The photo diode 52 performs a photoelectric conversion with an incident light and outputs an electric signal corresponding to the incident light. The electric signal responses to the change of the amount of hemoglobin in the blood vessel of the object person to be measured. Therefore, the pulse can be known from the output signal from the photo diode 52 and the arithmetic part 32 is capable of calculating the pulse rate per unit time in association with the result of the count of timer 34. The pulse sensor 50, for example, is preferably worn on an ear lobe or a fingertip of the object person to be measured.

The measurement of the pulse may be performed by combining a light-emitting device other than the LED and a light-receiving device other than the photodiode. In addition, the measurement on the basis of a pressure system, for example, may be employed instead of an optically measuring method. Alternatively, it is also possible to measure the heart beat of the object person to be measured using a heart rate meter configured to acquire the number of heart beats directly in a breast area and perform a process described later in the same manner as the pulse acquired by a pulse meter.

As shown in Fig. 1, the controller 40 is electrically connected to the operating unit 21, the display unit 22, the power source unit 23, the acceleration sensor 31, the arithmetic part 32, the memory 33, the timer 34, the A/D converter 35, and the pulse sensor 50 so that the operations of the respective members are controlled by the controller 40. The arithmetic part 32 and the controller 40 each are preferably formed by an integrated circuit.

The controller 40 functions as an instruction issuing unit configured to issue an instruction to the object person to be measured to execute a predetermined state. This instruction is issued by causing the display unit 22 to display a content of the predetermined state. Examples of the contents of the predetermined state to be displayed on the display unit 22 mainly include the type of action and the strength of action. Examples of the types of action include walking, running, resting, and standstill, and examples of the strengths of action include walking slowly, power walking, and slow running. One of examples of the display of the strength of action is, in the case of walking for example, to display images which change regularly so as to match the pace of walking on the display unit 22.

The arithmetic part 32 (an arithmetic unit) calculates a consumed energy consumed by the body movement of the object person to be measured under the control of the controller 40 on the basis of the biological data (for example, age, sex, body weight, and fat free mass) or the body movement data (the acceleration value) entered by the object person to be measured stored in the memory 33. Calculation of the consumed energy is performed by cumulatively adding the consumed energy of the body movement data at every unit time (for example, 20 seconds). In this manner, in this embodiment, the consumed energy measuring unit mainly includes the acceleration sensor 31, the A/D converter 35, and the arithmetic part 32.

The arithmetic part 32 also functions as a biological data calculating unit. The arithmetic part 32 is capable of defining a relationship between the pulse and the consumed energy, that is, a relational expression of the consumed energy with respect to the predetermined pulse rate on the basis of the measurement by the pulse sensor 50 and the measurement by the consumed energy measuring unit. By defining the relational expression of the consumed energy with respect to the predetermined pulse rate, calculation of the biological data as described below as an example on the basis of the relational expression is enabled.

The biometric apparatus 10 is preferably configured specifically as a small apparatus. For example, a body case (not shown) is configured to have a size which is accommodated in a chest pocket or the like. Arranged in the interior of the body case are the power source unit 23, the acceleration sensor 31, the arithmetic part 32, the memory 33, the timer 34, the A/D converter 35 and the controller 40, and provided on an outer surface of the body case are the operating unit 21 and the display unit 22. The pulse sensor 50 is configured to be capable of being unwound and wound by a cord reel provided in the interior of the body case. In this configuration, an apparatus which is easy to transport, convenient, and achieves simple measurement is obtained.

### [First Embodiment]

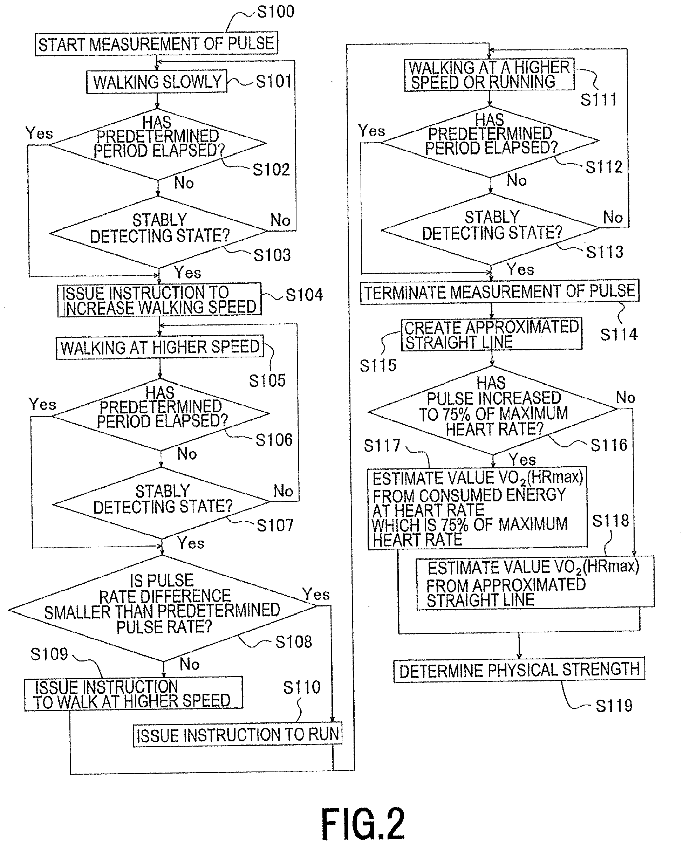

Referring now to Fig. 2, a first embodiment of the biometric apparatus 10 will be described. The arithmetic part 32 is capable of determining the physical strength of the object person to be measured. In the invention, the term "physical strength" means "endurance". Generally, the larger the endurance value VO₂max, the better it is evaluated to be. However, in the invention, in order to alleviate the load applied to the object person to be measured, the value VO₂ (that is, VO₂(HRmax)) at the maximum heart rate (pulse rate) close to the value VO₂max is calculated to use as a base of determination of the physical strength. The value VO₂ has a high correlation with a consumed energy and, simultaneously, the value VO₂(HRmax) also has a high correlation with a consumed energy (EE(75%HRmax)) at a heart rate (pulse rate) which is 75% of the maximum heart rate (pulse rate). The value EE(75%HRmax) may be estimated from the consumed energy and the heart rate (pulse rate) at the time of predetermined action. In other words, the determination of the body strength according to the invention is performed by estimating the value EE(75%HRmax) by measuring the pulse rate in a predetermined state and the consumed energy in the predetermined state, then acquiring the value VO₂(HRmax), and determining and evaluating the acquired value VO₂(HRmax).

In the example shown in Fig. 2, instructions of execution of three predetermined states (a first state, a second state, and a third state) for the object person to be measured are issued, and the physical strength is measured on the basis of the pulse rates and the consumed energies in the respective states. However, the number of the predetermined states to be issued as an instruction of execution may be two, or four or more according to the required measurement accuracy.

The pulse sensor 50 is attached to a predetermined position (for example, the ear lobe) of the object person to be measured, and measurement of the pulse is started (Step S100). The measurement of the pulse is started by the object person to be measured by performing a predetermined operation (such as pushing a start button) with respect to the operating unit 21. The controller 40 causes the pulse sensor 50 to start the measurement of the pulse and causes the timer 34 to start measurement of the elapsed time. The measurement of the pulse is performed continuously while the predetermined state is executed (up to Step S114).

Subsequently, the controller 40 issues an instruction to the object person to be measured to execute "walking slowly" as the first state, and then the object person to be measured performs a slow walking (Step S101). The issue of an instruction is performed, for example, by causing the display unit 22 to display the content of the instruction or by a voice instruction from a voice generating unit, not shown (the same goes for the description given below). The controller 40 causes the arithmetic part 32 to calculate the consumed energies at constant intervals (for example, 20 seconds) according to the result of detection obtained by the acceleration sensor 31 while the object person to be measured is walking, and causes the memory 33 to store the calculated result.

The controller 40 determines whether or not the predetermined period of execution has elapsed from the start of the walking (the first state) (Step S102). The period of execution may be, for example, four minutes, but is not limited specifically, and other periods may be set as needed. If the predetermined period of execution has elapsed from the start of the walking (the first state) (Yes in Step S102), the controller 40 issues an instruction to terminate the execution of the first state and issues an instruction to the object person to be measured to perform "walking at a higher speed" as the second state (Step S104). In other words, the second state is a state of action applied with a load larger than the first state.

If the predetermined period of execution is not elapsed from the start of the walking (the first state) (No in Step S102), the controller 40 determines whether or not the current state is a stably detecting state (Step S103). The determination whether or not it is the stably detecting state may be achieved by determining whether or not the fact that the change of the pulse rate of the object person to be measured in the first state falls within a predetermined range is detected and, for example, the determination may be achieved on the basis of whether or not the substantially same pulses have continued by twenty times. However, other conditions may be used for the determination.

If the state is determined to be the stably detecting state(Yes in Step S103), the controller 40 issues an instruction to terminate the execution of the first state before the elapse of the predetermined period of execution, and the procedure goes to Step S104. In contrast, if the state is determined not to be the stably detecting state (No in Step S103), the controller 40 causes the object person to be measured to continue the first state (walking slowly) (Step S101), and the procedures in Step S102 to Step S103 are repeated in the same manner as described above.

In this manner, even before the elapse of the predetermined period of execution (four minutes in the example above) from the start of the walking (the first state), if the state is the stably detecting state, reduction of period to execute the instructed first state is allowed by terminating the first state and transferring the instruction to an instruction to execute the second state, so that the load applied to the object person to be measured may be alleviated.

If the controller 40 issues an instruction to the object person to be measured to walk at a higher speed (the second state) (Step S104), the object person to be measured follows the instruction and performs the walking at a higher speed than the walking in the first state (see Step S101) (Step S105). In the walking of this state, the controller 40 causes the arithmetic part 32 to calculate the consumed energies at the constant intervals according to the result of detection obtained by the acceleration sensor 31 while the object person to be measured is walking, and causes the memory 33 to store the calculated result.

The controller 40 determines whether or not the predetermined period of execution has elapsed from the start (see Step S105) of the walking at a higher speed (the second state) (Step S106). The period of execution may be, for example, four minutes, but other periods may be set as needed in the same manner as in Step S102.

If the predetermined period of execution from the start of the walking at a higher speed has elapsed (the second state) (Yes in Step S106), the procedure goes to Step S108. In contrast, if the predetermined period of execution is not elapsed from the start of the walking at a higher speed (the second state) (No in Step S106), the controller 40 determines whether or not the current state is a stably detecting state (Step S107). The determination of the stably detecting state may be executed by determining whether or not the fact that the change of the pulse rate of the object person to be measured in the second state falls within a predetermined range within the period of continuous execution of the second state is detected in the same manner as in Step S103.

If the current state is determined to be the stably detecting state (Yes in Step S107), the controller 40 issues an instruction to terminate the execution of the second state before the elapse of the predetermined period of execution, and the procedure goes to Step S108. In contrast, if the current state is not determined to be the stably detecting state (No in Step S107), the controller 40 causes the object person to be measured to continue the current second state (walking at a higher speed) (Step S105), and the procedures in Step S106 to Step S107 are repeated in the same manner as described above.

In Step S108, the controller 40 determines whether or not the difference between the pulse rate in the first state (Step S101) and the pulse rate in the second state (Step S105) is a predetermined pulse rate (a predetermined threshold value) or smaller. The predetermined pulse rate may be set to nine pulses, for example, but may be other pulse rates.

If the pulse rate difference is larger than the predetermined pulse rate (No in Step S108), the controller 40 issues an instruction to the object person to be measured to perform "walking at a further higher speed" as the third state (Step S109). In contrast, if the pulse rate difference is the predetermined pulse rate or smaller in Step S108 (Yes in Step S108), the controller 40 issues an instruction to the object person to be measured to perform "running" as the third state (Step S110). If the difference between the pulse rate of the object person to be measured in the first state and the pulse rate of the object person to be measured in the second state is the predetermined threshold value or smaller, a state of action applying a load larger than that applied in the state of action instructed if the difference is larger than the predetermined threshold value is instructed as the third state. For reference sake, the third state is a state of action applied with a load larger than the second state.

As described thus far, "walking at a further higher speed" (Step S109) and "running" (Step S110) are selectively used as the third state depending on the difference between the pulse rate in the first state and the pulse rate in the second state. In other words, if there appears a little difference in the pulse rates between the first state and the second state because the object person to be measured has a high physical strength, an instruction to execute "running" is issued as the third state. In contrast, for the object person to be measured presenting the difference in the pulse rates between the first state and the second state is larger than the predetermined pulse rate, an instruction to "walk at a further higher speed" is issued as the third state. In this manner, by selectively using two types of the third states according to the physical strength of the object person to be measured, the object person to be measured is not forcedly applied with an excessive load, and an approximated straight line described later can be created with high degree of accuracy, whereby an accurate measurement of physical strength is achieved.

Subsequently, the object person to be measured performs the walking at a higher speed or the running according to the instruction to execute the third state in Step S109 or Step S110 (Step S111). The controller 40 causes the arithmetic part 32 to calculate the consumed energies at every predetermined period according to the result of detection obtained by the acceleration sensor 31 while the object person to be measured is walking or running, and causes the memory 33 to store the calculated result.

The controller 40 determines whether or not the predetermined period of execution has elapsed from the start of the walking at a higher speed or the running (the third state) (Step S112). The period of execution may be, for example, four minutes, other periods may be set in the same manner as in Step S102 and Step S106. If the predetermined period of execution has elapsed from the start of the walking at a higher speed or the running (the third state) (Yes in Step S112), the controller 40 issues an instruction to terminate the execution of the third state, and terminates the measurement of the pulse (Step S114).

If the predetermined period of execution has not elapsed from the start of the walking at a higher speed or the running (the third state) (No in Step S112), the controller 40 determines whether or not the current state is the stably detecting state (Step S113). The determination of the stably detecting state may be executed by determining whether or not the fact that the change of the pulse rate of the object person to be measured in the third state falls within a predetermined range within the period of continuous execution of the third state is detected in the same manner as in Step S103 and Step S107.

If the current state is determined to be the stably detecting state (Yes in Step S113), the controller 40 issues an instruction to terminate the execution of the third state before the elapse of the predetermined period of execution, and the measurement of the pulse is terminated (Step S114). In contrast, if the current state is determined not to be the stably detecting state (No in Step S113), the controller 40 causes the object person to be measured to continue the current third state (Step S111), and the procedures in Step S112 to Step S113 are performed in the same manner as described above.

After having terminated the measurement of the pulse (Step S114), the controller 40 creates an approximated straight line on the basis of the pulse rates and the consumed energies acquired respectively in the first state, the second state, and the third state, described above (Step S115). The creation of the approximated straight line corresponds to the definition of the relational expression between the pulse rates and the consumed energies acquired by the measurements.

Since there is a high correlation between the consumed energy and the pulse rate, in Step S115, the approximated straight line indicating the relationship therebetween is created. In this example, since the pulse rates and the consumed energies in three points in total, namely, in the first state, in the second state, and in the third state, as described above, the three points may be plotted by making a graph representing the consumed energy on a vertical axis and the pulse rate on a lateral axis. Therefore, the approximated straight line may be produced using the least square method on the basis of these three points.

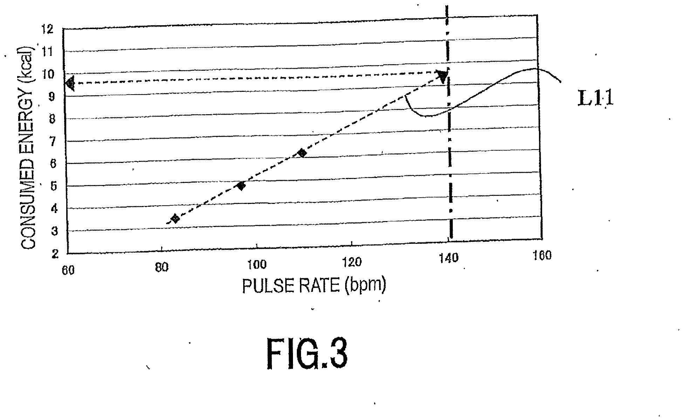

Fig. 3 to Fig. 5 are graphs made by plotting the pulse rates and the consumed energies in the first, second and third states and exemplifying the relationship thereof. Fig. 3 shows respective points when the three points corresponding to the first, second, and third states are moderately dispersed, and an approximated straight line L11 created on the basis of these three points.

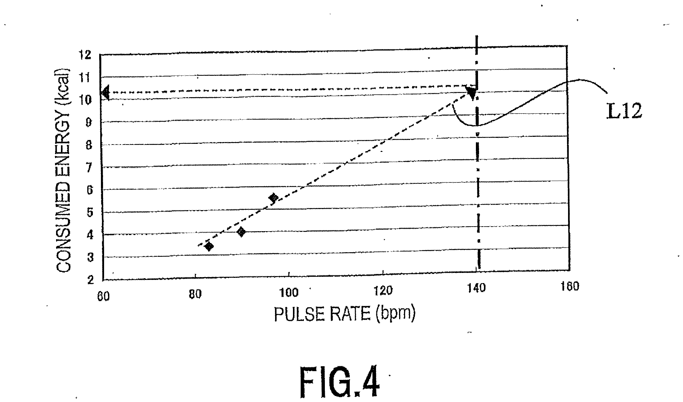

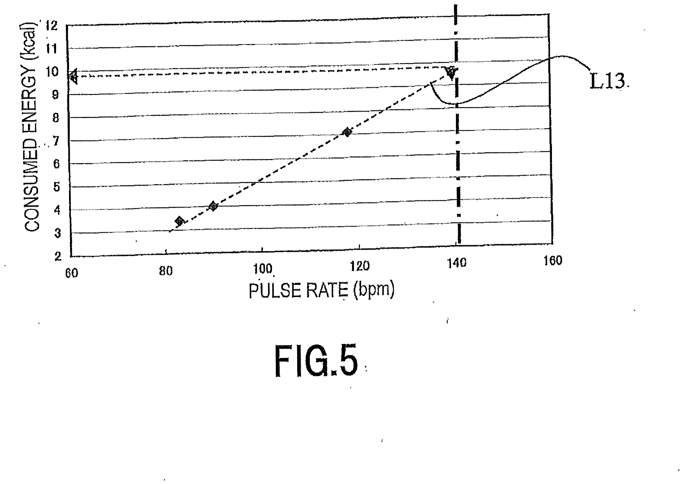

Referring now to Fig. 4 and Fig. 5, the significant of steps from Step S108 to Step S110 in Fig. 2 will be described. Fig. 4 shows a plot and an approximated straight line L12 in a case where the differences in the pulse rates in the first, second, and third states are small. For example, in a case where the object person to be measured has a high physical strength and hence the pulse rate may hardly be quickened even by performing some exercise, the difference between the pulse in the first state and the pulse in the second state may not be changed significantly. In such a case, on the condition that the instruction to "walk at a higher speed" is issued as the third state (see Step S109 in Fig. 2), in the same manner, the pulse in the third state may not be changed significantly. Therefore, even when the approximated straight line L12 (see Fig. 4) is acquired on the basis of the pulses and the consumed energies in the first state to the third state obtained in this manner, there is a high possibility that the approximated straight line L12 includes a large extent of uncertainty. In this case, estimation of EE (75%HRmax) performed later is also affected.

Therefore, if the difference between the pulse rates in the first state and the second state is small as in Step S108 in Fig. 2 (Yes in Step S108 in Fig. 2), an instruction to execute an action applying a higher load, namely, "running" as the third state (Step S110 in Fig. 2) is issued (Step S110 in Fig. 2) to change the pulse rate significantly. Fig. 5 shows the plot and an approximated straight line L13 in a case where the difference in the pulse rate in the first and second states is small, but the difference in the pulse rates between the second state and the third state is increased by issuing an instruction to run as the third state. As shown in Fig. 5, the point corresponding to the third state is dispersed from the two points corresponding to the first and second states. Accordingly, the approximated straight line including less uncertainty may be created.

After having performed the creation of the approximated straight line (Step S115), the controller 40 determines whether or not the pulse rate in either one of the first, second, and third states is increased to 75% of the maximum heart rate (pulse rate) (Step S116). Here, the maximum heart rate (pulse rate) may be calculated as "220 - (minus) age of the object person to be measured".

If the pulse rate in either one of the first, second, and third states is increased to 75% of the maximum heart rate (pulse rate) (Yes in Step S116), the controller 40 estimates VO₂(HRmax) on the basis of the consumed energy (EE(75%HRmax)) actually measured when the heart rate (pulse rate) is increased to 75% of the maximum heart rate (pulse rate) (Step S117). Step S116 and Step S117 are steps provided because determination of the physical strength with higher accuracy is enabled by using the actual measure value if the consumed energy (EE(75%HRmax)) obtained when the heart rate (pulse rate) is increased to 75% of the maximum heart rate (pulse rate). In contrast, the value VO₂(HRmax) may be estimated from the approximated straight line by Step S118 without providing Step S116 and Step S117 for simplifying the process. Step S115 for creating the approximated straight line may be performed only if it is determined to be No in Step S116 instead of being performed before Step S116.

If the pulse rate in either one of the first, second, and third states is not increased to 75% of the maximum heart rate (pulse rate) (No in Step S116), the controller 40 estimates the consumed energy (EE(75%HRmax)) may be obtained if the heart rate (pulse rate) is 75% of the maximum heart rate (pulse rate) on the basis of the approximated straight line created in Step S115, and estimates the value VO₂(HRmax) on the basis of the consumed energy (EE(75%HRmax)) (Step S118). In an example shown in Fig. 3, a case of the object person to be measured of 30 years old is shown, and the heart rate (pulse rate), which is 75% of the maximum heart rate (pulse rate), becomes 142.5 bpm (see alternate long and short dash line in the drawing). The controller 40 estimates (calculates) the value VO₂(HRmax) on the basis of the consumed energy (EE(75%HRmax)) at the pulse rate of 142.5 bmp (Step S118).

Estimation (calculation) of the value VO₂(HRmax) on the basis of EE(75%HRmax) performed in Step S117 and Step S118 is performed on the basis of a predetermined regression expression (an expression representing a correlation between the value EE(75%HRmax) and the value VO₂(HRmax)). As described above, the value VO₂(HRmax) has a high a correlation with the consumed energy (EE(75%HRmax)) at a heart rate (pulse rate) which is 75% of the maximum heart rate (pulse rate). Therefore, the regression expression may be set as an expression representing the correlation between the value EE(75%HRmax) and the value VO₂(HRmax). For example, the regression expression may be set by collecting sample data relating to the value VO₂(HRmax) at the value EE(75%HRmax) aimed at an indefinite number of subjects in advance, plotting the data in a graph having a vertical axis representing EE(75%HRmax) (EE(75%HRmax)/kg) per 1 kg of weight and a lateral axis representing the oxygen uptake (VO₂(HRmax)/kg) at the maximum heart rate (pulse rate) per 1 kg of weight, and obtaining the approximated straight line thereof.

The controller 40 determines the physical strength of the object person to be measured from the value VO₂(HRmax) estimated (calculated) in Step S117 or Step S118 (Step S119). Although the contents of the determination of the physical strength may be set as needed, the determination may be performed by providing determination tables relating to the value VO₂(HRmax) classified by age spans for male and female and applying the calculated value VO₂(HRmax) as described above into the corresponding determination table as an example. The contents of the determination in the determination table may be classified by age spans such as "19 years old or younger", "20 to 24 years old", "25 to 29 years old", ... "65 to 69 years old", and "70 years old or older", each of the age span being divided into five stages in predetermined value spans of VO₂(HRmax), namely, "very low", "low", "average", "good", and "very good" by setting the average value of the value VO₂(HRmax) of each age span at the center. The result of determination in Step S119 is displayed on the display unit 22.

In the configuration as described above, the following advantages are achieved according to the above-described embodiment.
(1) Since the physical strength may be determined only by performing walking or running arbitrarily, the object person to be measured is not obliged to perform a heavy exercise for a long time, and hence the determination of the physical strength without applying a large load to the object person to be measured is achieved.
(2) The physical strength may be determined using a consumed energy on the basis of the result of detection from the acceleration sensor, and hence usage of a large scale of apparatus is not necessary, and the determination in a site of daily life is enabled.
(3) The relational expression of the consumed energy with respect to the predetermined pulse rate determined by the invention is defined on the basis of data specifically on each of the object persons to be measured, and hence the determination of the physical strength with high degree of accuracy is enabled.
(4) What is necessary is just to perform the measurement of a plurality of the states of action respectively for a short time, and hence a simple measurement is achieved.
(5) What is necessary is just to perform a light exercise such as resting or walking according to the instruction given by the apparatus, and hence the measurement may be performed without an assistant who measures the time of exercise or issues an instruction about paces of exercise.

### [Modification of First Embodiment]

The biometric apparatus 10 includes the operating unit 21 as the data input unit which allows input of age, sex, body weight, and fat free mass of the object person to be measured. Specifically, the fat free mass entered via the operating unit 21 relates strongly to muscle mass, and hence the large fat free mass means the large muscle mass. Therefore, in the first embodiment described above, the controller 40 as the instruction issuing unit may be configured to issue an instruction to execute a state of action applying a larger load than the state of action to be instructed when the fat free mass shows a predetermined reference value or smaller as the second state and/or the third state if the entered fat free mass is larger than the predetermined reference value. In this configuration, for the object person to be measured having a large fat free mass (and thus the large muscle mass), the pulse rate can be increased within a moderate range by issuing an instruction to execute the state of action with higher load as the second state and/or the third state, whereby the approximated straight line can be acquired with higher degree of accuracy.

### [Second Embodiment]

Subsequently, a second embodiment of the invention will be described. The second embodiment is different from the first embodiment in that an instruction to execute a resting state in the upright position is issued as the first state. Other procedures and the configuration of the biometric apparatus 10 used in the measurement of physical strength are the same as those in the first embodiment. Therefore, detailed description about the same configurations as those of the biometric apparatus 10 in the first embodiment will be omitted. For the reference sake, it is needless to say that the resting states other than in the upright position, for example, the state of lying or the state of sitting on a chair may be employed as the first state.

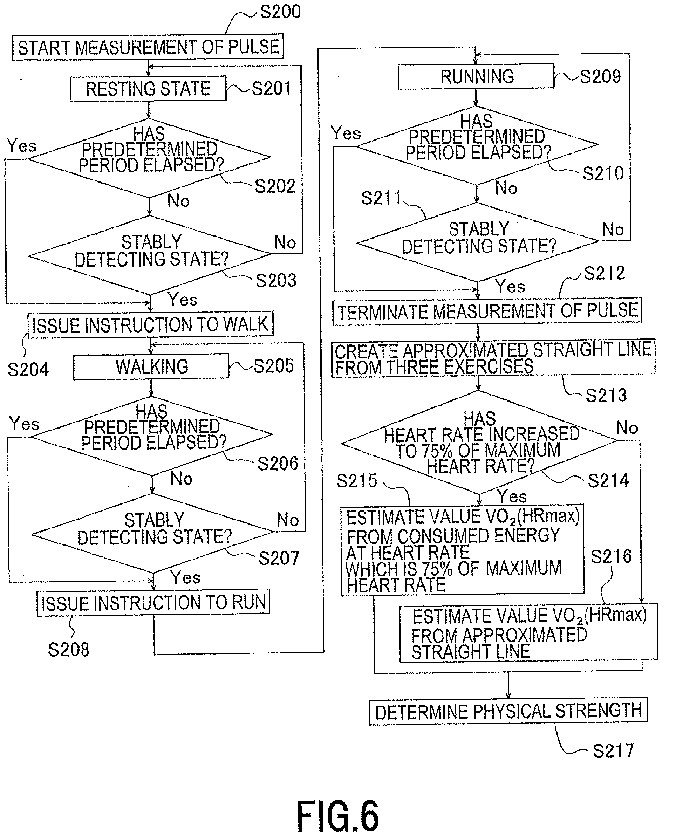

Fig. 6 is a flowchart indicating a flow of the measurement of physical strength according to the second embodiment of the invention. In the example shown in Fig. 6, instructions of execution of three predetermined states for the object person to be measured are issued, and the physical strength of the object person to be measured is measured on the basis of the pulse rates and the consumed energies in the respective predetermined states. However, the number of the predetermined states to be issued as instructions of execution may be two, or four or more according to the required measurement accuracy.

The pulse sensor 50 is attached to the predetermined position (for example, the ear lobe) of the object person to be measured, and the measurement of the pulse is started (Step S200). The measurement of the pulse is started by the object person to be measured by performing the predetermined operation (such as pushing the start button) with respect to the operating unit 21. The controller 40 causes the pulse sensor 50 to start the measurement of the pulse and causes the timer 34 to start measurement of the elapsed time. The measurement of the pulse is performed continuously while the predetermined state is executed (up to Step S212).

Subsequently, the controller 40 issues an instruction to the object person to be measured to execute a "maintaining the resting state" (for example, resting in the upright position) as the first state, and then the object person to be measured performs the resting state (Step S201). The controller 40 causes the arithmetic part 32 to calculate the consumed energies at the constant intervals (for example, 20 seconds) on the basis of the result of detection obtained by the acceleration sensor 31 while the object person to be measured is in the resting state, or the basal metabolic rate calculated from the entered biological data such as age, sex, and body weight and causes the memory 33 to store the calculated result.

The controller 40 determines whether or not the predetermined period of execution has elapsed from the start of the resting state (the first state) (Step S202). If the predetermined period of execution has elapsed from the start of the resting state (the first state) (Yes in Step S202), the controller 40 issues an instruction to terminate the execution of the first state and issues an instruction to the object person to be measured to perform "walking" as the second state (Step S204). In other words, the second state is a state of action applied with a load larger than the first state.

If the predetermined period is not elapsed from the start of the resting state (the first state) (No in Step S202), the controller 40 determines whether or not the current state is the stably detecting state (Step S203). The determination of whether or not it is the stably detecting state may be executed by determining whether or not the fact that the change of the pulse rate of the object person to be measured in the first state falls within a predetermined range within the period of continuous execution of the first state is detected.

If the state is determined to be the stably detecting state (Yes in Step S203), the controller 40 issues an instruction to terminate the execution of the first state before the elapse of the predetermined period of execution, and the procedure goes to Step S204. In contrast, if the state is not the stably detecting state (No in Step S203), the controller 40 causes the object person to be measured to continue the current first state (the resting state) (Step S201), and the procedures in Step S202 to Step S203 are repeated in the same manner as described above.

In this manner, even before the elapse of the predetermined period of execution (four minutes, for example) from the start of the resting state (the first state), if the state is determined to be the stably detecting state, reduction of period to perform the instructed first state is allowed by terminating the first state and transferring the instruction to an instruction to execute the second state, so that the load applied to the object person to be measured may be alleviated.

If the controller 40 issues an instruction to the object person to be measured to perform the walking (the second state) (Step S204), the object person to be measured performs the walking according to the instruction (see Step S205). In the walking of this state as well, the controller 40 causes the arithmetic part 32 to calculate the consumed energies at the constant intervals according to the result of detection obtained by the acceleration sensor 31 while the object person to be measured is walking, and causes the memory 33 to store the calculated result.

The controller 40 determines whether or not the predetermined period of execution has elapsed from the start (Step S205) of the walking (the second state) (Step S206).

If the predetermined period of execution from the start of the walking (the second state) has elapsed (Yes in Step S206), the procedure goes to Step S208. In contrast, if the predetermined period of execution from the walking (the second state) is not elapsed (No in Step S206), the controller 40 determines whether or not the current state is the stably detecting state (Step S207). The determination of the stably detecting state may be executed in the same manner as Step S203.

If the current state is determined to be the stably detecting state (Yes in Step S207), the controller 40 issues an instruction to terminate the execution of the second state before the elapse of the predetermined period of execution, and the procedure goes to Step S208. In contrast, if the state is determined not to be the stably detecting state (No in Step S207), the controller 40 causes the object person to be measured to continue the current second state (walking) (Step S205), and the process in Step S206 to Step S207 are repeated in the same manner as described above.

If the controller 40 issues an instruction to the object person to be measured to perform the running (the third state) (Step S208), the object person to be measured performs the running according to the instruction (Step S209). In this running state as well, the controller 40 causes the arithmetic part 32 to calculate the consumed energies at the constant intervals according to the result of detection obtained by the acceleration sensor 31 while the object person to be measured is running, and causes the memory 33 to store the calculated result.

The controller 40 determines whether or not the predetermined period of execution has elapsed from the start (see Step S209) of the running (the third state) (Step S210). If the predetermined period of execution has elapsed from the start of running (third state) (Yes in Step S210), the controller 40 terminates the measurement of the pulse (Step S212).

If the predetermined period of execution is not elapsed from the start of running (third state) (No in Step S210), the controller 40 determines whether or not the current state is the stably detecting state (Step S211). The determination of the stably detecting state may be executed in the same manner as Step S203.

If the state is determined to be the stably detecting state (Yes in Step S211), the controller 40 issues an instruction to terminate the execution of the third state before the elapse of the predetermined period of execution, and the measurement of the pulse is terminated (Step S212).

In contrast, if the state is determined not to be the stably detecting state (No in Step S211), the controller 40 causes the object person to be measured to continue the current third state (Step S209), and the processes in Step S210 to Step S211 are repeated in the same manner as described above.

After having terminated the measurement of the pulse (Step S212), the controller 40 creates an approximated straight line on the basis of the pulse rates and the consumed energies acquired respectively in the first state, the second state, and the third state, described above (Step S213). The creation of the approximated straight line corresponds to the definition of the relational expression between the pulse rate and the consumed energy acquired by the measurements. A method of creating the approximated straight line is the same manner as the first embodiment.

After having created the approximated straight line (Step S213), the controller 40 determines whether or not the pulse rate in either one of the first, second, and third states is increased to 75% of the maximum heart rate (pulse rate) (Step S214). If the pulse rate in either one of the first, second, and third states is increased to 75% of the maximum heart rate (pulse rate) (Yes in Step S214), the controller 40 estimates VO₂(HRmax) on the basis of the consumed energy (EE(75%HRmax)) actually measured if the heart rate (pulse rate) is increased to 75% of the maximum heart rate (pulse rate) (Step S215).

If the pulse rate in either one of the first, second, and third states is not increased to 75% of the maximum heart rate (pulse rate) (No in Step S214), the controller 40 estimates the consumed energy (EE(75%HRmax)) obtained if the heart rate (pulse rate) is 75% of the maximum heart rate (pulse rate) on the basis of the approximated straight line created in Step S213, and estimates (calculates) the value VO₂(HRmax) on the basis of the consumed energy (EE(75%HRmax)) (Step S216).

The controller 40 determines the physical strength of the object person to be measured from the value VO₂(HRmax) estimated (calculated) in Step S215 or Step S216 (Step S217).

According to the second embodiment, an instruction to execute the first state is given as the resting state, and hence the determination of the physical strength with a small load for the object person to be measured may be performed. Other configurations, actions and effects are the same as those in the first embodiment.

### [Third Embodiment]

Subsequently, a third embodiment of the invention will be described. In the third embodiment, an instruction to execute a state of action executed continuously for a predetermined period in the same content is issued as a predetermined state, and the controller 40 as the biological data calculating unit acquires an approximated straight line on the basis of the pulse rate and the consumed energy of the object person to be measured in the predetermined state measured by a plurality of times during the predetermined period, so that a relational expression of the consumed energy with respect to the predetermined pulse rate of the object to be measured is defined. In other words, the predetermined state in the third embodiment is different from those in the first embodiment and the second embodiment in that an instruction to execute a single type of state of action is issued as the predetermined state continuously for a predetermined period, and in that the relational expression is defined on the basis of a plurality of points of measurement acquired in the predetermined period.

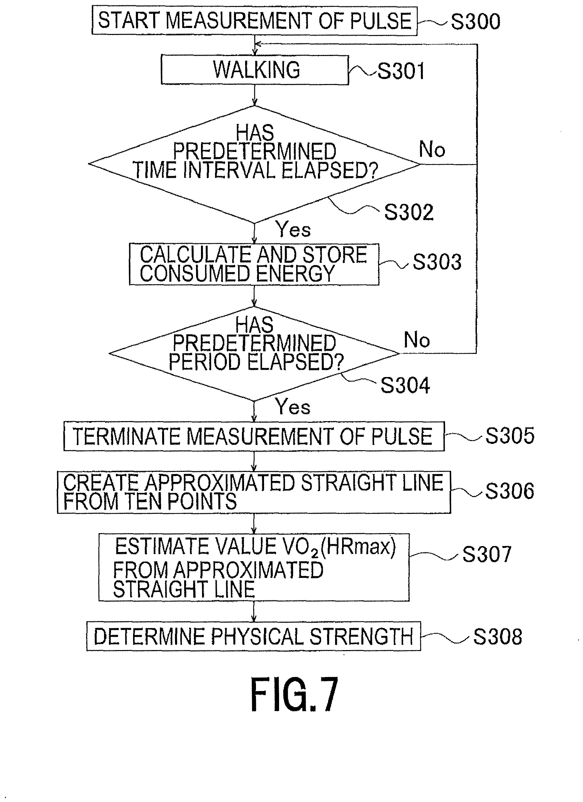

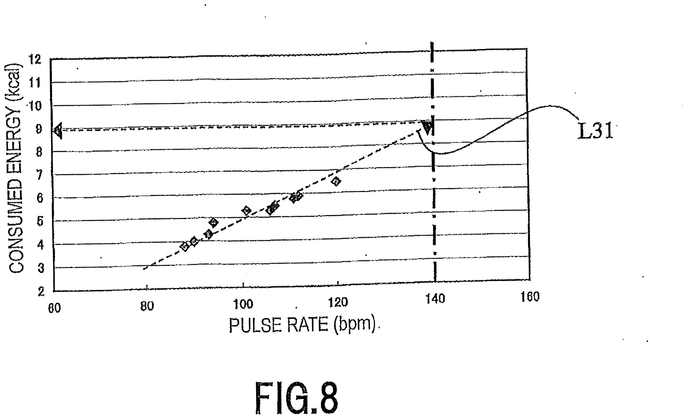

Fig. 7 is a flowchart showing a flow of the measurement of physical strength according to the third embodiment of the invention, and Fig. 8 is a graph showing a relationship between the pulse rate and the consumed energy plotting the pulse rate and the consumed energy measured by a plurality of times within the predetermined period.

The pulse sensor 50 is attached to the predetermined position (for example, the ear lobe) of the object person to be measured, and measurement of the pulse is started (Step S300). The measurement of the pulse is started by the object person to be measured by performing the predetermined operation (such as pushing the start button) with respect to the operating unit 21. The controller 40 causes the pulse sensor 50 to start the measurement of the pulse and causes the timer 34 to start measurement of the elapsed time. The measurement of the pulse is performed continuously while the predetermined state is executed (up to Step S305).

Subsequently, the controller 40 issues an instruction to the object person to be measured to perform the walking as the predetermined state, and then the object person to be measured performs the walking (Step S301).

The controller 40 determines whether or not the predetermined time interval has elapsed from the start of the walking (Step S302). The predetermined time interval may be one minute, for example, and may be set to other period.

If the predetermined time interval is not elapsed from the start of the walking (No in Step S302), the controller 40 causes the object person to be measured to continue the walking as the predetermined state (Step S301).

If the predetermined time interval has elapsed from the start of the walking (Yes in Step S302), the controller 40 causes the arithmetic part 32 to calculate the consumed energy in the predetermined time interval according to the result of detection by the acceleration sensor 31 in the predetermined time interval, and causes the memory 33 to store the calculated consumed energy (Step S303).

Subsequently, the controller 40 determines whether or not the predetermined period has elapsed from the start of the walking for the first time (Step S304). This predetermined time may be 10 minutes, for example, and may be set to other time period.

While the period from the start of the walking for the first time does not reach the predetermined period, (No in Step S304), the procedures from Step S301 to Step S303 are repeatedly performed. Accordingly, measurement of the pulse rate and the consumed energy of the object person to be measured in the predetermined state may be performed at every elapse of the predetermined time interval (one minute) during the predetermined period (10 minutes). The measurement during the predetermined period (10 minute) is not necessarily limited to be performed in the same time intervals, and what is required is only to perform the measurement by an arbitrary plurality of times during the predetermined period (10 minutes).

If it is determined that the period from the start of the walking for the first time reaches the predetermined period (Yes in Step S304), the controller 40 issues an instruction to terminate the execution of the predetermined state, and terminates the measurement of the pulse rate (Step S305).

After having terminated the measurement of the pulse (Step S305), the controller 40 creates an approximated straight line on the basis of the pulse rate and the consumed energy of the object person to be measured in the predetermined state measured by a plurality of times during the predetermined period (Step S306, Fig. 8). The creation of the approximated straight line corresponds to the definition of the relational expression between the pulse rate and the consumed energy acquired by the measurements. Fig. 8 shows respective points corresponding to ten measuring points and an approximated straight line L31 created on the basis of the measuring points. Even by the execution of the same predetermined state (walking), the pulse rate is increased gradually by continuing for the predetermined period (10 minutes), the plurality of the measuring points measured within the predetermined period (10 minutes) may be acquired by a state of being dispersed as shown in Fig. 8. The approximated straight line L31 may be acquired in the same manner as the method of creating the approximated straight line in the first embodiment on the basis of these measuring points.

The controller 40 estimates the value VO₂(HRmax) from the approximated straight line created in Step S306 in the same manner as the first embodiment (Step S307), and determines the physical strength of the object person to be measured from the estimated value VO₂(HRmax) (Step S308). The determination of the physical strength is the same as that in the first embodiment.

According to the third embodiment, the determination of the physical strength may be performed only by issuing an instruction to perform a single type of predetermined state and causing the object person to be measured to perform the single type of predetermined state continuously for a predetermined period, an extremely simple determination of the physical strength can be performed. Other configurations, actions, and effects are the same as those in the first embodiment.

### [Fourth Embodiment]

Subsequently, a fourth embodiment of the invention will be described. In the fourth embodiment, in the same manner as the first embodiment to the third embodiment (including the modification) described above, a relational expression of the consumed energy with respect to the predetermined pulse rate of the object person to be measured is defined, and using this relational expression, the consumed energy (biological data) of the object person to be measured is calculated.

The arithmetic part 32 defines a relationship between the pulse and the consumed energy (a relational expression of the consumed energy with respect to the predetermined pulse rate) on the basis of the measurement by the pulse sensor 50 and the measurement by the consumed energy measuring unit. What is required is to define the relational expression of the consumed energy with respect to the predetermined pulse rate of the object person to be measured using any one of methods from Step S100 to Step S115 in Fig. 2 in conjunction with the first embodiment, and from Step 200 to Step S213 in Fig. 6 in conjunction with the second embodiment, from Step S300 to Step S307 in Fig. 7 in conjunction with the third embodiment. There is a high correlation between the pulse and the consumed energy. Therefore, after having acquired the approximated straight line L11 as shown in Fig. 3 or the approximated straight line L31 as shown in Fig. 8 as a relational expression of the consumed energy with respect to the predetermined pulse rate, which is specific for the object person to be measured, the consumed energy consumed at the corresponding period can be calculated easily by measuring the pulse rate of the object person to be measured and applying the measured pulse rate to the relational expression (approximated straight line). Therefore, the calculation of the consumed energy performed by detecting the body movement of the object person to be measured using the acceleration sensor does not necessarily have to be performed. Therefore, in the state of action which cannot be calculated accurately by a method of obtaining the magnitude of the acceleration value of the body movement by the acceleration sensor and calculating the consumed energy as in the related art, for example, in the state of operating a bicycle or climbing a mountain, the consumed energy can be calculated accurately by measuring the pulse rate at the corresponding period. Since both of the pulse sensor 50 and the acceleration sensor 31 are provided, if the measurement of the pulse by the pulse sensor 50 is performed, the consumed energy may be obtained as described above on the basis of the pulse rate, and if the measurement of the pulse by the pulse sensor 50 is not performed, the consumed energy may be obtained from the acceleration value of the body movement as in the related art.

Although the invention has been described on the basis of the above-described embodiments, the invention is not limited to the above-described embodiments, and can be improved or modified within the scope of the object of the improvement and the spirit of the invention.

### Industrial Applicability

As described above, the biometric apparatus and the method of measuring the physical strength according to the invention is suitable for the application for measuring the physical strength easily and confirming the state of health.

## Claims

1. A biometric apparatus comprising:
an instruction issuing unit configured to issue an instruction to an object person to be measured to execute a predetermined state;
a pulse sensor configured to measure a pulse rate in the predetermined state;
a consumed energy measuring unit configured to measure a consumed energy in the predetermined state; and
a biological data calculating unit configured to define a relational expression of the consumed energy with respect to a predetermined pulse rate of the object person to be measured on the basis of the pulse rate measured by the pulse sensor in the predetermined state and the consumed energy measured by the consumed energy measuring unit at the pulse rate, and calculate biological data of the object person to be measured on the basis of the relational expression.

2. The biometric apparatus according to Claim 1,
wherein the instruction issuing unit issues an instruction to execute at least a first state, a second state which is a state of action applying a load larger than that of the first state, and a third state which is a state of action applying a load larger than that of the second state in sequence as the predetermined state, and the biological data calculating unit defines the relational expression by acquiring an approximated straight line on the basis of three points, namely, a pulse rate and a consumed energy of the object person to be measured in the first state, a pulse rate and a consumed energy of the object person to be measured in the second state, and a pulse rate and a consumed energy of the object person to be measured in the third state.

3. The biometric apparatus according to Claim 2,
wherein if the difference between the pulse rate of the object person to be measured in the first state and the pulse rate of the object person to be measured in the second state is a predetermined threshold value or smaller, the instruction issuing unit issues an instruction to execute a state of action applying a load larger than that applied in the state of action to be instructed if the difference is larger than the predetermined threshold value as the third state.

4. The biometric apparatus according to any one of Claims 1 to 3, further comprising a data input unit configured to be capable of entering data such as age, sex, body weight, and lean body mass of the object person to be measured, wherein the consumed energy measuring unit includes an acceleration sensor configured to be capable of detecting an acceleration value of a body movement of the object person to be measured, and calculates the consumed energy on the basis of the acceleration value of the body movement detected by the acceleration sensor and the data such as age, sex, body weight, and lean body mass entered by the data input unit.

5. The biometric apparatus according to Claim 4,
wherein if the lean body mass entered by the data input unit is larger than a predetermined reference value, the instruction issuing unit issues an instruction to execute a state of action applying a load larger than that of the state of action to be instructed if the lean body mass is a predetermined reference value or smaller as the second state and/or the third state.

6. The biometric apparatus according to any one of Claims 1 to 5, wherein if the fact that the change of the pulse rate of the object person to be measured in the predetermined state falls within a predetermined range is detected during the period of execution in which the predetermined state is continuously executed, the instruction issuing unit issues an instruction to terminate the execution of the predetermined state before the elapse of the period of execution, and if the fact is not detected, the instruction issuing unit issues an instruction to terminate the execution of the predetermined state after the period of execution has elapsed.

7. The biometric apparatus according to Claim 1,
wherein the instruction issuing unit issues an instruction to execute a state of action executed continuously for a predetermined period in the same content as the predetermined state, and the biological data calculating unit defines the relational expression by acquiring the approximated straight line on the basis of the pulse rate and the consumed energy of the object person to be measured in the predetermined state measured by a plurality of times during the predetermined period.

8. The biometric apparatus according to any one of Claims 1 to 7, wherein the biological data calculating unit calculates a value EE(75%HRmax) of the object person to be measured on the basis of the relational expression as the biological data, and calculates a value VO₂(HRmax) of the object person to be measured by applying the value EE(75%HRmax) to an expression expressing a correlation between the value EE(75%HRmax) and the value VO₂(HRmax).

9. The biometric apparatus according to Claim 8,
wherein the biological data calculating unit determines the physical strength of the object person to be measured from the value of VO₂(HRmax) of the object person to be measured, the sex and the age of the object person to be measured, and displays the result of determination on a display unit.

10. The biometric apparatus according to any one of Claims 1 to 9, wherein the biological data calculating unit calculates the consumed energy according to the pulse rate by applying the pulse rate of the object person to be measured acquired by the pulse sensor to the relational expression as the biological data.
